(19) Europäisches Patentamt
European Patent Office
Office européen des brevets

(11) Numéro de publication: **0 394 122 A2**

(12) # DEMANDE DE BREVET EUROPEEN

(21) Numéro de dépôt: **90401041.0**

(22) Date de dépôt: **18.04.90**

(51) Int. Cl.5: **A61K 31/65**

(30) Priorité: **19.04.89 FR 8905195**

(43) Date de publication de la demande: **24.10.90 Bulletin 90/43**

(84) Etats contractants désignés: **AT BE CH DE DK ES FR GB GR IT LI LU NL SE**

(71) Demandeur: **RHONE-POULENC SANTE**
**20, avenue Raymond Aron**
**F-92165 Antony Cédex(FR)**

(72) Inventeur: **Lemaitre, Marc**
**1bis, Avenue Jean-Jaurès**
**F-78000 Versailles(FR)**
Inventeur: **Zerial, Aurélio**
**3, Allée de l'Orme à Piquets**
**F-94350 Villiers sur Marne(FR)**

(74) Mandataire: **Savina, Jacques et al**
**RHONE-POULENC SANTE, Service Brevets,**
**20 Avenue Raymond Aron**
**F-92165 Antony Cédex(FR)**

(54) **Application de tétracyclines pour obtenir un médicament destiné au traitement du sida et à sa prévention.**

(57) Application d'une tétracycline de formule générale (I) dans laquelle :
- $R_1$ et $R_2$ représentent des atomes d'hydrogène ou des radicaux hydroxy ou méthyle ou forment ensemble un radical méthylène,
- $R_3$ représente un radical carbamoyle éventuellement substitué par un reste pyrrolidinyl-1 méthyle ou (amino-5 carboxy-5 pentylamino) méthyle,
- $R_4$ représente un atome d'hydrogène ou un radical diméthylamino et
- $R_5$ représente un atome d'hydrogène ou un radical hydroxy,
à l'état libre ou le cas échéant sous forme de sel ou d'hydrate pour obtenir un médicament destiné au traitement ou à la prévention du SIDA ou de syndromes analogues.

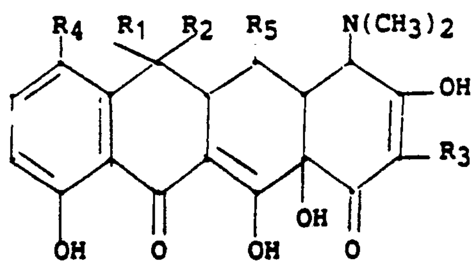

EP 0 394 122 A2

Xerox Copy Centre

# APPLICATION DE TETRACYCLINES POUR OBTENIR UN MEDICAMENT DESTINE AU TRAITEMENT DU SIDA ET A SA PREVENTION

La présente invention concerne l'obtention d'un médicament utile pour la prophylaxie et le traitement thérapeutique du SIDA (syndrome d'immunodéficience acquise) et de syndromes associés [ARC (AIDS related complex)]. Par prophylaxie nous sous-entendons le traitement de sujets qui ont été exposés aux virus HIVs (human immunodeficiency virus), en particulier les séropositifs asymptomatiques, qui présentent le risque de développer la maladie dans les mois et les années à venir après la primoinfection.

Selon l'invention, ce médicament contient un ou plusieurs produits de la classe des tétracyclines et répondant à la formule générale :

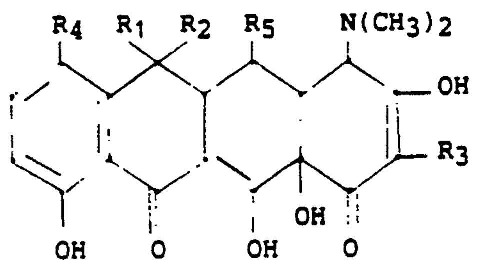

(I)

dans laquelle :

- $R_1$ et $R_2$ représentent des atomes d'hydrogène ou des radicaux hydroxy ou méthyle ou forment ensemble un radical méthylène,
- $R_3$ représente un radical carbamoyle éventuellement substitué par un reste pyrrolidinyl-1 méthyle ou (amino-5 carboxy-5 pentylamino) méthyle,
- $R_4$ représente un atome d'hydrogène ou un radical diméthylamino et
- $R_5$ représente un atome d'hydrogène ou un radical hydroxy,

à l'état libre ou le cas échéant sous forme de sel.

On a trouvé que les produits de formule générale (I) inhibent l'effet cytopathogène du virus HIV en cultures cellulaires à des concentrations dépourvues d'effet cytostatique. Cet effet est sélectif à l'égard des cellules infectées.

Il a également été trouvé que les cellules ainsi protégées de l'effet cytopathogène, arrêtent la production du virus après environ 2 mois de culture.

La présente invention concerne l'obtention d'une composition pharmaceutique capable de maintenir en vie les cellules infectées par le virus HIV et d'arrêter la production virale à long terme et donc de réduire la progression vers le SIDA ou de diminuer sa gravité chez les sujets déjà infectés.

Ladite composition est administrable par voie parentérale, rectale, intralymphatique ou orale. Dans le cas de l'administration par voie orale la dose journalière chez l'homme sera comprise entre 0,1 et 4 g. Ces doses sont bien tolérées et donnent lieu à des pics de concentrations sériques qui sont au moins dix fois supérieures aux concentrations minimales actives anti-HIV. Un autre avantage de ces produits est lié à leur longue demi-vie dans le sérum. Leur temps de demi-vie est en effet de plusieurs heures, le plus souvent proche de 18 heures.

Dans ce qui suit on décrit les résultats de ces produits sur l'effet cytopathogène du virus HIV dans une lignée cellulaire permissive (cellules lymphoblastoïdes humaines CEM) ainsi que dans les lymphocytes humains des cellules sanguines périphériques.

## ACTIVITE DE TETRACYCLINES VIS-A-VIS DE L'EFFET CYTOPATHOGENE DU VIRUS HIV EN CULTURES CELLULAIRES

Le test est réalisé sur la lignée lymphoblastoïde CEM clone 13. Dans une microplaque de 96 puits on dépose 25 µl/puits d'une solution de produit à tester ou de tampon phosphate isotonique (TPI) dans le cas des contrôles. Les produits sont étudiés à différentes concentrations (souvent 8), à raison de 4 puits par concentration. On ajoute alors 125 µl d'une suspension de cellules CEM dans le milieu RPMI contenant 10

% de sérum de veau foetal, 40 U/ml de pénicilline, 40 µg/ml de streptomycine et 5 µg/ml de polybrène (3500 cellules par puits) et les microplaques sont incubées une heure à 37° C, sous une atmosphère contenant 5 % de gaz carbonique. Pour chaque concentration, l'essai est partagé en 2 parties : une partie sur cellules infectées, pour la détermination de l'activité antivirale et l'autre partie sur cellules non infectées, pour déterminer la cytotoxicité des produits. On infecte alors la première série avec HIV (100 µl par puit d'une suspension de virus LAV-1-BRU contenant 200-300 TCID50) tandis que l'autre série reçoit 100 µl de milieu RPMI tel que défini précédemment. Au bout de 7 jours d'incubation, 175 µl de surnageant sont prélevés et rejetés. La viabilité cellulaire est déterminée selon une modification de la technique décrite par R. Pauwels et coll., J. Virol. Meth., 20, 309-321 (1988).

Les puits, contenant encore les cellules (infectées ou non infectées), reçoivent 10 µl d'une solution de MTT (méthylthiazoletétrazolium) à 5 mg/ml dans du tampon phosphate isotonique. Pendant une période d'incubation de 4 heures, le MTT est converti en un sel de formazan (bleu) à l'intérieur des cellules vivantes et proportionnellement à leur nombre, alors que rien n'apparaît dans les puits ne contenant plus que des cellules mortes. On ajoute alors 120 µl d'isopropanol (contenant 0,04 mole/l d'acide chlorhydrique) et les microplaques sont agitées jusqu'à la solubilisation du bleu de formazan. L'absorption à 540 nm est lue avec un lecteur automatique de réactions ELISA en microplaques.

Pour les essais de plus longue durée, on procède comme décrit précédemment jusqu'au jour 7. A ce moment, 100 µl de cellules sont prélevés pour mesurer la proportion de cellules vivantes et le restant est remis en culture avec du milieu frais contenant les produits aux mêmes concentrations. Cette opération est répétée tous les 4 jours.

Les résultats obtenus au jour 7 sont donnés dans le tableau I ci-après.

Les essais sont poursuivis jusqu'aux jours 9, 14 et 18. Le tableau II montre les résultats obtenus aux jours 14 et 18 : l'activité protectrice de l'effet cytopathogène est maintenue.

TABLEAU I

| Exemple | $R_1$ | $R_2$ | $R_3$ | $R_4$ | $R_5$ | Concentration µg/ml | Cellules infectées Densité optique | Cellules non infectées Densité optique |
|---|---|---|---|---|---|---|---|---|
| 1 (tétracycline) | $-CH_3$ | $-OH$ | $-CONH_2$ | $-H$ | $-H$ | 0 | 0,360 | 1,000 |
| | | | | | | 0,03 | 0,350 | 1,025 |
| | | | | | | 0,08 | 0,350 | 1,080 |
| | | | | | | 0,25 | 0,340 | 1,050 |
| | | | | | | 0,74 | 0,420 | 1,100 |
| | | | | | | 2,22 | 0,510 | 1,040 |
| | | | | | | 6,67 | 0,620 | 0,900 |
| | | | | | | 20 | 0,400 | 0,460 |
| | | | | | | 60 | 0,1 | 0,1 |
| 2 (doxycycline) | $-H$ | $-CH_3$ | $-CONH_2$ | $-H$ | $-OH$ | 0 | 0,820 | 1,550 |
| | | | | | | 0,01 | 0,820 | 1,530 |
| | | | | | | 0,04 | 0,785 | 1,34 |
| | | | | | | 0,12 | 0,720 | 1,53 |
| | | | | | | 0,37 | 1,000 | 1,60 |
| | | | | | | 1,11 | 1,240 | 1,50 |
| | | | | | | 3,33 | 1,370 | 1,50 |
| | | | | | | 10 | 0,660 | 0,740 |
| | | | | | | 30 | 0,200 | 0,2 |
| 3 (métacycline) | $=CH_2$ | | $-CONH_2$ | $-H$ | $-OH$ | 0 | 0,590 | 1,350 |
| | | | | | | 0,01 | 0,570 | 1,100 |
| | | | | | | 0,04 | 0,640 | 1,380 |
| | | | | | | 0,12 | 0,680 | 1,300 |
| | | | | | | 0,37 | 0,730 | 1,230 |
| | | | | | | 1,11 | 0,780 | 1,180 |
| | | | | | | 3,33 | 1,060 | 1,320 |
| | | | | | | 10 | 0,770 | 0,960 |
| | | | | | | 30 | 0,1 | 0,1 |
| 4 (lymecycline) | $-CH_3$ | $-OH$ | $-CONHCH_2NH(CH_2)_4$<br>$H_2N-CH-COOH$ | $-H$ | $-H$ | 0 | 0,8 | 1,65 |
| | | | | | | 0,01 | 0,75 | 1,75 |
| | | | | | | 0,04 | 1,25 | 1,80 |
| | | | | | | 0,12 | 1,21 | 1,90 |
| | | | | | | 0,37 | 1,20 | 1,75 |
| | | | | | | 1,11 | 1,28 | 1,80 |
| | | | | | | 3,33 | 1,40 | 1,85 |
| | | | | | | 10 | 1,55 | 1,85 |
| | | | | | | 30 | 1,05 | 1,05 |
| 5 (minocycline) | $-H$ | $-H$ | $-CONH_2$ | $-N(CH_3)_2$ | $-H$ | 0 | 0,750 | 1,550 |
| | | | | | | 0,01 | 0,660 | 1,590 |
| | | | | | | 0,04 | 0,720 | 1,650 |
| | | | | | | 0,12 | 0,890 | 1,750 |
| | | | | | | 0,37 | 1,210 | 1,670 |
| | | | | | | 1,11 | 1,510 | 1,760 |
| | | | | | | 3,33 | 1,340 | 1,650 |
| | | | | | | 10 | 0,450 | 0,480 |
| | | | | | | 30 | 0,320 | 0,420 |

Les résultats des produits des exemples 2 et 5, obtenus au jour 7, sont illustrés par la figure 1.

TABLEAU II

| | | JOUR 14 | | JOUR 18 | |
|---|---|---|---|---|---|
| | Concentration du produit étudié µg/ml | Cellules infectées Densité optique | Cellules non infectées Densité optique | Cellules infectées Densité optique | Cellules non infectées Densité optique |
| Doxycycline produit de l'exemple 2 | 0 | 0,250 | 1,000 | 0,200 | 1,050 |
| | 0,01 | 0,250 | 0,980 | 0,240 | 1,100 |
| | 0,04 | 0,300 | 0,950 | 0,275 | 1,050 |
| | 0,12 | 0,450 | 1,020 | 0,250 | 1,020 |
| | 0,37 | 0,550 | 1,000 | 0,300 | 1,000 |
| | 1,11 | 0,880 | 0,960 | 0,700 | 1,020 |
| | 3,33 | 0,750 | 0,950 | 0,750 | 0,950 |
| | 10 | 0,100 | 0,100 | 0,100 | 0,100 |
| Minocycline produit de l'exemple 5 | 0 | 0,250 | 1,050 | 0,200 | 1,100 |
| | 0,01 | 0,300 | 1,100 | 0,250 | 1,050 |
| | 0,04 | 0,450 | 1,250 | 0,250 | 1,050 |
| | 0,12 | 0,620 | 1,200 | 0,450 | 1,100 |
| | 0,37 | 1,250 | 1,250 | 1,000 | 1,150 |
| | 1,11 | 1,250 | 1,200 | 0,950 | 1,100 |
| | 3,33 | 0,400 | 0,350 | 0,350 | 0,400 |
| | 10 | 0,100 | 0,100 | 0,100 | 0,100 |

Dans une autre expérience, des cellules CEM protégées de l'effet cytopathogène du virus HIV, par 1,2 µg/ml de minocycline (produit de l'exemple 5) ou de doxycycline (produit de l'exemple 2), sont cultivées à partir du jour 18 après l'infection, dans des flacons de culture de 25 cm3. Chaque échantillon est divisé en 2 parties, l'une recevant à nouveau le produit étudié et l'autre cultivée en l'absence de produit.

Les cellules sont cultivées pendant 2 mois.

Les 2 séries de culture restent protégées vis-à-vis de l'effet cytopathogène du virus (même en l'absence de produit).

En conclusion, l'effet cytoprotecteur apporté par les produits de formule générale (I) entre le jour 7 et le jour 18, est maintenu indéfiniment.

Il ressort de ces essais que les tétracyclines de formule générale (I) exercent un effet protecteur contre l'effet cytopathogène du virus HIV à des concentrations dépourvues de toute toxicité sur cellules saines (non infectées). Cette cytoprotection est persistante après suppression des produits. Il est très important de rappeler que ces concentrations actives sont facilement atteintes dans le plasma de patients après administration de ces médicaments à des posologies approuvées par la pharmacopée et donc non toxiques.

## DETERMINATION DE LA REVERSE TRANSCRIPTASE

Les cellules infectées traitées ou non de manière continue par la minocycline ou la doxycycline ont été examinées en ce qui concerne la production de virus. La technique utilisée pour la détermination virale a été celle du dosage de la reverse transcriptase du virus HIV.

L'activité de la reverse transcriptase est mesurée directement sur 50 µl de surnageant de culture. 10 µl de tampon contenant 0,5 M de KCl, 5 mM de dithiotreitol (DTT) et 0,5 % de Triton X-100 sont ajoutés à tous les micropuits contenant les 50 µl de surnageant à tester. 40 µl de tampon contenant 1,25 mM d'éthylène glycol-bis($\beta$-aminoéthyl éther) (EGTA), 0,125 M de Tris.HCl pH 7,8, 12,5 mM de $MgCl_2$, 3 µCi de ($^3H$) Thymidine triphosphate (TTP), 0,05 $DO_{260}$ d'acide polyadénylique - acide thymidylique (poly rA-oligo dT) sont ajoutés ensuite. La microplaque est couverte au moyen d'un film plastique et incubée 60 minutes à 37° C.

On arrête la réaction en ajoutant 20 µl d'une solution glacée de 120 mM de $Na_4P_2O_7$ dans 60 % d'acide trichloracétique et les échantillons sont placés 15 minutes sur un lit de glace.

Les précipités sont filtrés sur fibres de verre en utilisant un laveur de cellules (Skatron) et les filtres sont lavés avec une solution de 12 mM de $Na_4P_2O_7$ dans 5 % d'acide trichloracétique.

Les filtres sont séchés et la radioactivité est comptée après ajout d'un liquide scintillant. Les essais sont faits en triple.

Les résultats obtenus sont rassemblés dans le tableau III ci-après. La figure 2 illustre ces résultats.

Entre 20 et 50 jours après l'infection, une quantité importante [20 000 à 37 000 coups par minute (cpm)] de reverse transcriptase est décelée dans les surnageants. A partir du jour 53, la production de reverse transcriptase diminue pour arriver à des valeurs négligeables quelques jours après.

En conclusion, les cellules protégées, entre les jours O et 18 par les produits selon l'invention, cessent leur production de virus HIV à partir du jour 56.

TABLEAU III

| | Activité de la reverse transcriptase coups/mn x $10^{-3}$ | Jour après infection |
|---|---|---|
| Minocycline (présente du jour 0 au jour 18) | 27<br>21<br>37<br>19<br>21<br>17<br>3<br>2<br>3 | 20<br>39<br>42<br>46<br>49<br>53<br>56<br>60<br>80 |
| Doxycycline (présente du jour 0 au jour 18) | 21<br>30<br>31<br>21<br>24<br>14<br>2<br>3<br>2 | 20<br>39<br>42<br>46<br>49<br>53<br>56<br>60<br>80 |
| Minocycline (présente du jour 0 à la fin de l'expérienc-e) | 25<br>33<br>33<br>29<br>37<br>14<br>2<br>2<br>5 | 20<br>39<br>42<br>46<br>49<br>53<br>56<br>60<br>80 |
| Doxycycline (présente du jour 0 à la fin de l'expérienc-e) | 25<br>26<br>31<br>27<br>18<br>10<br>2<br>1,4<br>2 | 20<br>39<br>42<br>46<br>49<br>53<br>56<br>60<br>80 |

Les tétracyclines selon l'invention entraînent à long terme un arrêt de la production de virus par les cellules.

INHIBITON DE L'EFFET CYTOPATHOGENE DU VIRUS HIV EN CULTURE DE LYMPHOCYTES HUMAINS

Préparation de la suspension cellulaire

La source des leucocytes humains est un stock congelé en $N_2$ liquide (100 x $10^6$ cellules/ampoule) de leucocytes obtenus sur cytaphérèse d'individus (donneurs) sains. Au moment de l'essai, les cellules sont décongelées, mises en suspension dans 80 % de sérum de veau foetal (SVF) et 20 % de diméthylsulfoxyde et centrifugées à 1000 rpm (10 minutes). Le culot est mis en suspension dans un milieu RPMI [contenant 10 % Lymphocult (Interleukine 2 en provenance de Biotest Diagnostic), 10 % de SVF, pénicilline et streptomycine, respectivement 40 U/ml et 40 µg/ml], à raison de 1 x $10^6$ cellules/ml. Habituellement, on utilise un flacon de culture cellulaire contenant 40 à 60 ml de cette suspension cellulaire. Les cellules sont incubées 4 jours en présence de phytohémagglutinine PHA (0,1 % DIFCO), à 37° C sous une atmosphère contenant 95 % d'air et 5 % de gaz carbonioue. Les cellules sont alors centrifugées, lavées une fois dans du milieu frais (ci-dessus) et remises en suspension dans le même milieu à 1 x $10^6$ cellules/ml [(RPMI 10 % de SVF, 10 % de Lymphocult-antibiotiques ci-dessus) mais contenant 10 U/ml d'anticorps anti-interferon (IFN α) 5 µg/ml de prolybrène.

Réalisation du test

200 µl de cette suspension cellulaire (200.000 cellules) sont déposés dans des puits d'une microplaque de cultures (à 96 puits). A chaque puits, on ajoute 25 µl du produit à tester [dilué 10 fois dans du tampon phosphate isotonique (TPI)] ou 25 µl du solvant TPI pour le contrôle. L'essai est fait en 2 séries : sur cellules infectées (pour déterminer l'activité antivirale) et sur cellules non infectées pour déterminer l'activité antiproliférative (cytotoxicité). On infecte alors avec 25 µl du virus HIV (souche LAV-BRU), alors que l'autre série reçoit (par puits) 25 µl du milieu (ci-dessus). Les cultures sont incubées 7 jours à 37° C (95 % d'air, 5 % de $CO_2$). 200 µl de surnageant sont alors prélevés, rejetés ou utilisés pour la détermination de l'activité transcriptase inverse. Les cellules du fond (sous 50 µl) sont alors soit testées pour leur viabilité (test MTT), soit additionnées de 200 µl de milieu frais (ci-dessus). Dans ce dernier cas, l'incubation est prolongée pendant encore 7 jours (test au jour 14). L'opération peut être répétée une semaine après (test au jour 21).

Les résultats obtenus 21 jours après infection sont indiqués dans le tableau IV et illustrés par la figure 3.

TABLEAU IV

| | Concentration du produit à tester µg/ml | Cellules infectées Densité optique | Cellules non infectées Densité optique |
|---|---|---|---|
| | 0 | 0,355 | 0,702 |
| Minocycline | 0,04 | 0,530 | 0,836 |
| | 0,12 | 0,435 | 0,775 |
| | 0,37 | 0,640 | 0,850 |
| | 1,11 | 0,835 | 0,980 |
| | 3,33 | 0,750 | 0,900 |
| | 10 | 0,540 | 0,833 |
| Doxycycline | 0,04 | 0,530 | 0,775 |
| | 0,12 | 0,590 | 0,765 |
| | 0,37 | 0,550 | 0,853 |
| | 1,11 | 0,773 | 0,835 |
| | 3,33 | non déterminée | 0,670 |
| | 10 | 0,125 | 0,103 |

Les dérivés de tétracycline de formule générale (I) peuvent exister à l'état de sels pharmaceutiquement acceptables.

Parmi ces sels peuvent notamment être cités les sels d'addition avec les acides organiques ou minéraux sels que notamment les chlorhydrates, bromhydrates, iodhydrates, sulfates, nitrates, phosphates, ou organiques tels que acétates, propionates, succinates, maléates, fumarates, citrates, tartrates, gluconates, adamantoates, méthanesulfonates, p.toluènesulfonates, iséthionates ou pénicillinates ou des dérivés de substitution de ces composés, ainsi que les sels avec les métaux alcalins tels que les sels de sodium, de potassium, de lithium, ou avec les métaux alcalino-terreux tel que les sels de calcium ou de magnésium, ou

tout autre sel pharmaceutiquement acceptable connu pour être utile dans le domaine des tétracyclines.

Il est entendu que les tétracyclines de formule générale (I) peuvent aussi exister à l'état d'hydrates et que ces hydrates entrent également dans le cadre de la présente invention.

La présente invention concerne donc l'obtention d'un médicament contenant au moins un produit de formule générale (I) sous forme libre ou sous forme de sel, ce (ou ces) produit(s) pouvant être à l'état pur ou sous forme d'une composition pharmaceutique en association avec un ou plusieurs diluants ou adjuvants compatibles et pharmaceutiquement acceptables.

Les compositions stériles pour administration parentérale peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou d'autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans un milieu stérile injectable.

Les compositions pour administration rectale sont les suppositoires ou les capsules rectales, qui contiennent outre le principe actif des excipients tels que le beurre de cacao, des glycérides semi-synthétiques ou des polyéthylèneglycols.

Les compositions administrables par voie intralymphatique sont préparées comme les compositions stériles pour administration parentérale. A titre d'exemple, par dissolution d'une quantité convenable d'une tétracycline de formule générale (I) dans un solvant stérile pour injection, tel qu'une solution aqueuse isotonique de chlorure de sodium ou de mannitol.

Comme compositions solides pour administration orale peuvent être utilisés des comprimés, des pilules, des poudres ou des granulés. Dans ces compositions, le produit actif selon l'invention (éventuellement associé à un autre produit pharmaceutiquement compatible) est mélangé à un ou plusieurs diluants ou adjuvants inertes, tels que saccharose, lactose ou amidon. Ces compositions peuvent également comprendre des substances autres que les diluants, par exemple un lubrifiant tel que le stéarate de magnésium.

Comme compositions liquides pour administration orale, on peut utiliser des émulsions pharmaceutiquement acceptables, des solutions, des suspensions, des sirops et des élixirs contenant des diluants inertes tels que l'eau ou l'huile de paraffine. Ces compositions peuvent également comprendre des substances autres que les diluants, par exemple des produits mouillants, édulcorants ou aromatisants.

Les exemples suivants illustrent des compositions contenant des produits de formule générale (I).

EXEMPLE A

On prépare des flacons de poudre stérile destinée à une administration par voie intra-veineuse et des ampoules de solvant, en effectuant la répartition suivante :

| | |
|---|---|
| - Flacon de poudre stérile : | |
| Minocycline sous forme de chlorhydrate ... | 100 mg |
| - Ampoule de solvant : | |
| Eau pour préparation injectable ..... | 5 cm3 |

EXEMPLE B

On prépare des flacons de poudre stérile destinée à une administration par voie intra-musculaire et des ampoules de solvant, en effectuant la répartition suivante :

| - Flacon de poudre stérile : | |
|---|---|
| Minocycline sous forme de chlorhydrate ... | 100 mg |
| - Ampoule de solvant : | |
| Chlorhydrate de lidocaïne .....<br>Gluconate de magnésium .....<br>Hydroxyde de sodium .....<br>Eau pour préparation injectable .... | 40 mg<br>15 mg<br>q.s.p. 6,5 < pH < 6,9<br>q.s.p. 3 cm3 |

EXEMPLE C

On prépare des gélules destinées à une administration par voie orale ayant la composition suivante :

Minocycline sous forme de chlorhydrate ... 100 mg

- Excipient :

Amidon de maïs
Stéarate de magnésium
Silice colloïdale

**Revendications**

1 - Application d'un produit appartenant à la classe des tétracyclines et répondant à la formule générale :

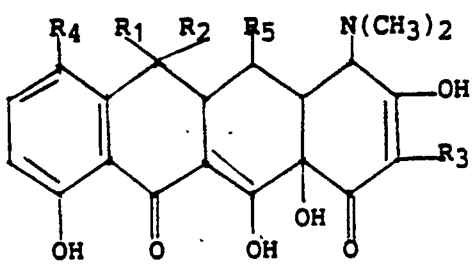

dans laquelle :
- $R_1$ et $R_2$ représentent des atomes d'hydrogène ou des radicaux hydroxy ou méthyle ou forment ensemble un radical méthylène,
- $R_3$ représente un radical carbamoyle éventuellement substitué par un reste pyrrolidinyl-1 méthyle ou (amino-5 carboxy-5 pentylamino) méthyle,
- $R_4$ représente un atome d'hydrogène ou un radical diméthylamino et
- $R_5$ représente un atome d'hydrogène ou un radical hydroxy,
à l'état libre ou le cas échéant sous forme de sel ou d'hydrate pour obtenir un médicament destiné au traitement ou à la prévention du SIDA ou de syndromes analogues.

2 - Procédé de préparation d'une composition pharmaceutique destinée au traitement ou à la prévention du SIDA ou de syndromes analogues, caractérisé en ce que l'on mélange au moins un produit de la classe des tétracyclines tel que défini dans la revendication 1, avec un ou plusieurs diluants ou adjuvants compatibles et pharmaceutiquement acceptables.

Figure 1

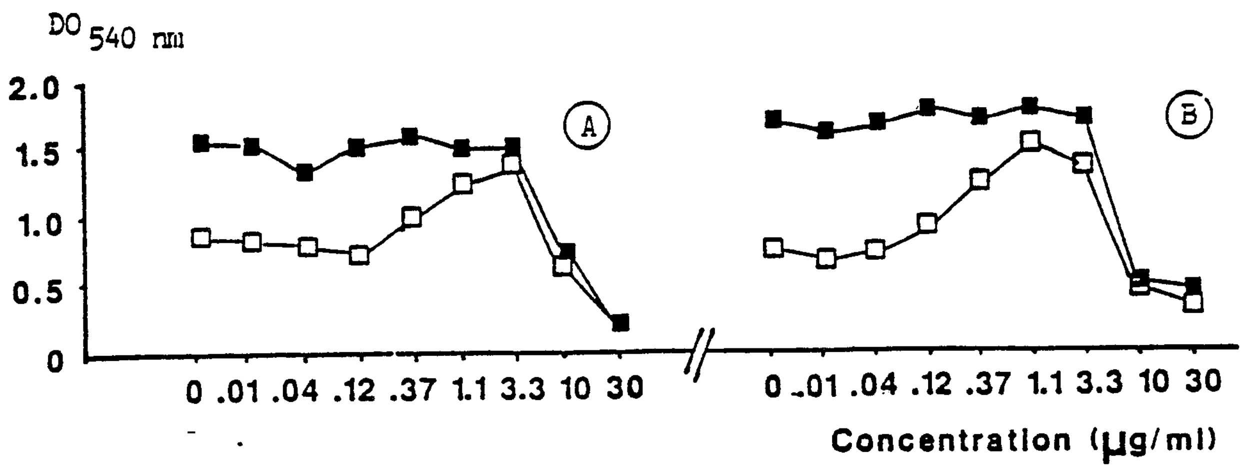


A = Doxycycline B = Minocycline

Jour 7 = Après infection

Activité de la doxycycline et de la minocycline dans les cellules CEM infectées (—□—) ou non infectées par HIV (—■—). La viabilité cellulaire est déterminée au jour 7 après l'infection par la méthode MTT.

Figure 2

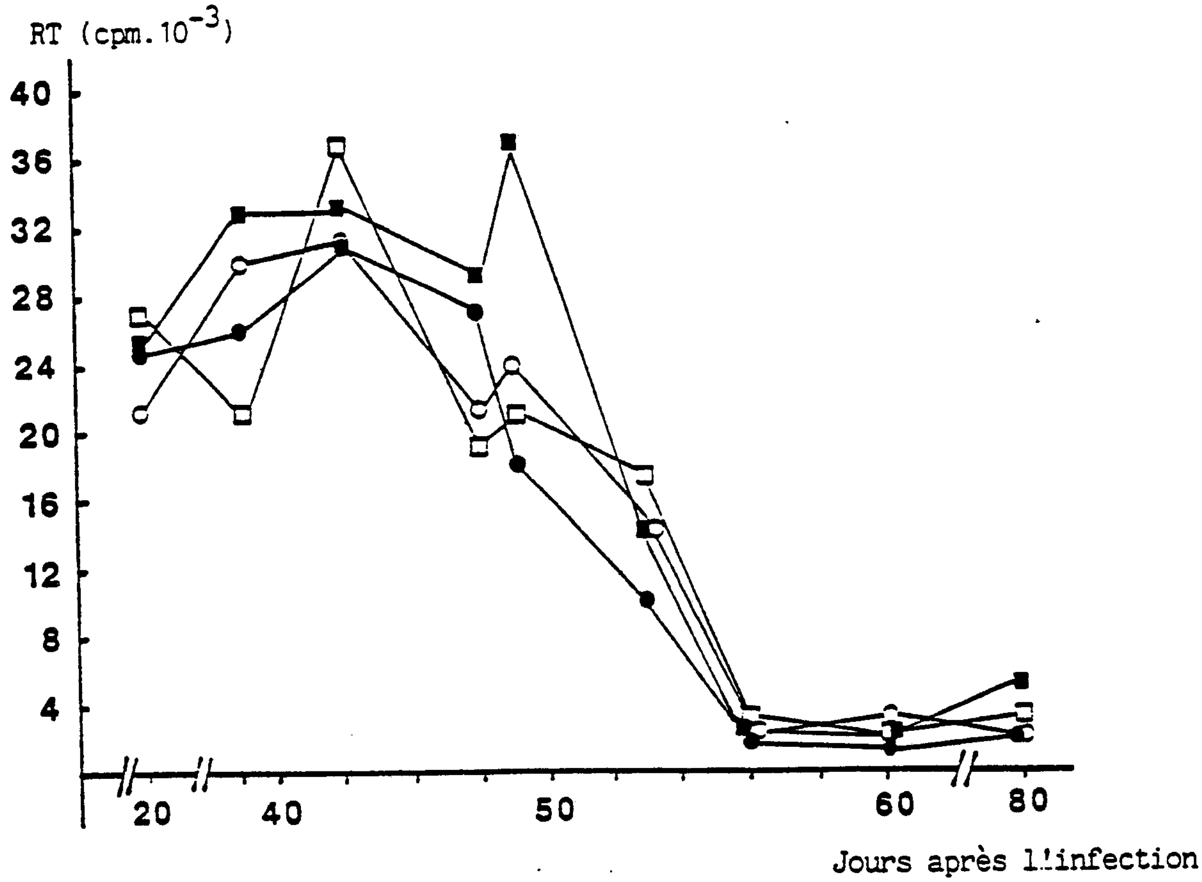


Activité de la reverse transcriptase dans les surnageants de cellules CEM obtenus à des temps différents par rapport à l'infection HIV (jour 0). La minocycline ou la doxycycline étaient présentes à raison de 1,1 µg/ml :

- du jour 0 au jour 18 (respectivement —□— et —○— )
- ou du jour 0 à la fin de l'expérience (respectivement —■— et —●— ).

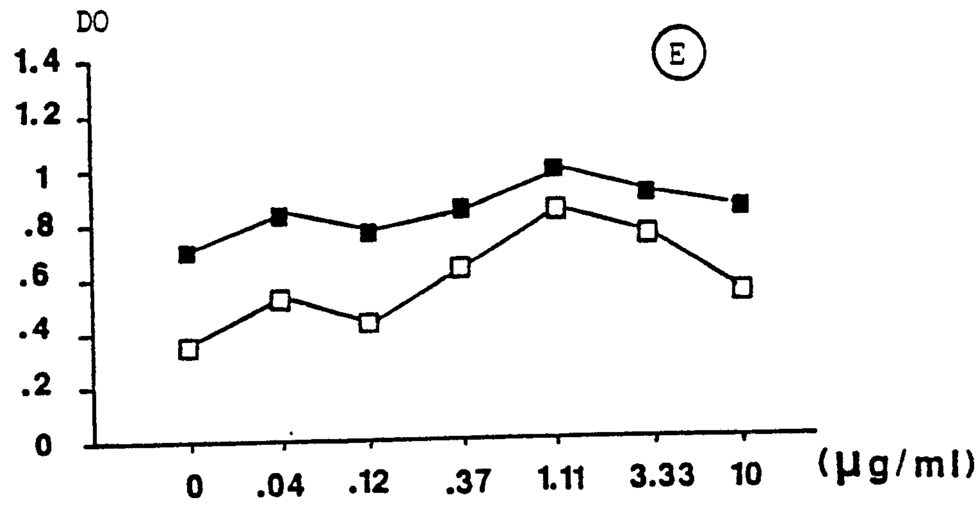


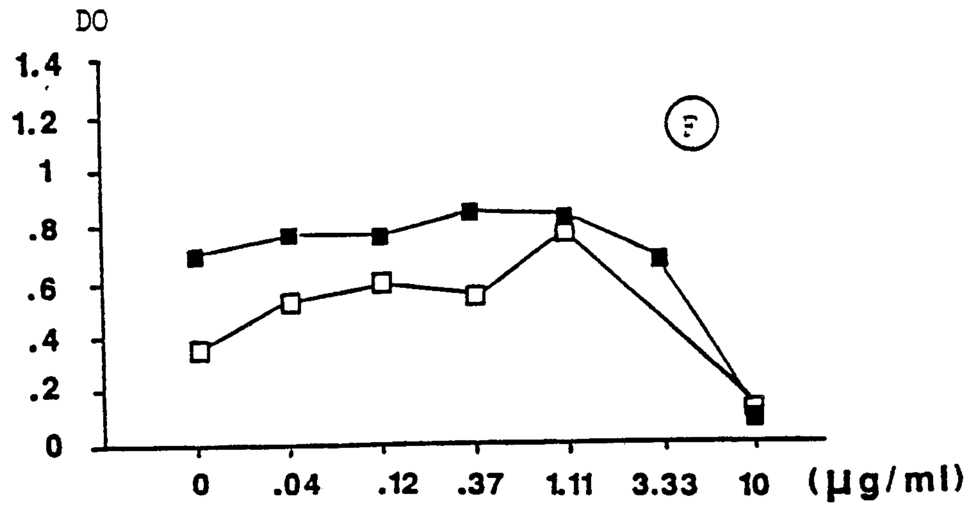


Figure 3

Activité de la minocycline (E) et doxycycline (F) sur lymphocytes humains au jour 21 après l'infection.